# EUROPEAN PATENT APPLICATION

(11) **EP 4 342 520 A1**
(43) Date of publication of application: **27.03.2024**
(21) Application number: 22825227.6
(22) Date of filing: 10.06.2022
(51) Int. Cl.: A61N 1/44, A61N 1/36, A61N 1/05, H05H 1/24, H05H 1/34

(54) **PLASMA GENERATION DEVICE FOR UTERINE CERVIX**

(30) Priority: 16.06.2021 KR 20210078303
(71) Applicant: IBMsol Co., Ltd., Seoul 02447 (KR)
(72) Inventor: KWON, Byung Su, Seoul 01879 (KR); KIM, Hee Kyung, Seoul 01879 (KR)
(74) Representative: BCKIP Part mbB
(86) International application number: PCT/KR2022/008185
(87) International publication number: WO 2022/265302

(57) **Abstract**

Disclosed is a plasma generation module for a uterine cervix, comprising: a module body coupled to the front end of a probe part capable of entering the vagina of the human body; a dielectric coupled to the outer front of the module body and facing the uterine cervix, while maintaining a gap for a plasma generation space between the module body and the uterine cervix; and an electrode between the module body and the dielectric and having a shape corresponding to a shape of the dielectric, wherein the dielectric includes a gas discharge part discharging background gas into the plasma generation space.

## Description

### [Technical Field]

The disclosure relates to a plasma generation module for a uterine cervix, and more particularly, to a plasma generation module for a uterine cervix that may concentrate plasma generation in both external and internal cervical canals of a uterine cervix transformation zone where a uterine cervix precancerous lesion occurs.

### [Background Art]

Cervical cancer may be the fourth most common female cancer worldwide. In 2012, 500,000 or more cases of the cancer were diagnosed worldwide, and approximately 50% of patients died from this disease.

In the United States, approximately 13,000 cases of invasive cervical cancer and approximately 4,100 cancer-related deaths are reported to occur every year.

The cervical cancer may be a disease that may be completely cured if detected early because the cervical cancer goes through a precancerous lesion stage for a longer time period than other cancers, and abnormalities in a cervix may be detected through a simple test rather than diagnostic test for stomach, lung, colon, and thyroid cancers. Upon considering these facts, early detection and active treatment of the uterine cervix precancerous lesion may be important

In Korea, while the cervical cancer has continuously decreased, the overall number of patients with the uterine cervix precancerous lesion has increased, thus rapidly increasing a frequency of medical use.

Currently, there is no treatment medicine for the uterine cervix precancerous lesion. The only treatment may be surgical resection, and surgical resection methods may include loop electrosurgical excision procedure (LEEP) and uterine cervix-cervical conization, laser excision, and hysterectomy.

However, these surgical treatments may cause social problems such as premature birth, miscarriage, infertility, and a resulting decrease in a birth rate, and there is a risk of recurrence in case of incomplete surgery. In addition, there is a risk of giving birth to a child with cerebral palsy, retinal disorders, or reduced lung maturity due to premature birth although its probability is not high.

As such, the uterine cervix precancerous lesion may cause a rapid increase in incidence at younger ages and serious pregnancy-related complications in conventional conization. Therefore, there is an urgent need for the development of new safe and effective treatments considering the current social situation of a low birth rate.

Meanwhile, among methods for treating cancer cells, various treatment methods other than the surgical treatment methods are being actively researched and developed currently. Among these methods, the development of a plasma treatment device that may kill the cancer cells without causing pain is being actively developed.

Atmospheric pressure plasma may be an ionized medium containing active ingredients including electrons and ions, free radicals, reactive molecules and photons, and may be classified as thermal plasma or non-thermal plasma.

In particular, the non-thermal atmospheric pressure plasma is emerging as a new tool in biomedical applications because this plasma may interact with a target biological material without causing thermal damage to a surrounding tissue. However, as disclosed in Korean Patent Nos. 10-1592081 and 10-1248668, there is no published technology for killing cervical cancer cells by using the non-thermal atmospheric pressure plasma. Therefore, there is an urgent need for the development of related technology because there is no published technology for killing the cervical cancer cells by using the non-thermal atmospheric pressure plasma.

It is necessary to apply a dielectric barrier discharge (DBD) plasma method which allows large-area plasma treatment with only a certain voltage and a small amount of background gas in order to simultaneously treat both external and internal cervical canals of a uterine cervix transformation zone where the uterine cervix precancerous lesion occurs. Plasma discharge using the DBD method may use a method in which the plasma is directly generated through resonance in a space between a dielectric surrounding an electrode and a target object to be treated. Therefore, it is important to maintain a certain distance between the dielectric and the target object to be treated. The uterine cervix of a human body has a unique shape of the exposed circular external cervical canal of the uterine cervix and its internal cervical canal that is connected to the inside of the center. Therefore, in order to effectively generate plasma energy, it is necessary to develop a plasma generation module having a special structure that reflects an anatomical structure of the uterine cervix and may maintain the certain distance between the plasma dielectric and the target object to be treated.

### [Related Art Document]

### [Patent Document]

Korean Patent No. 10-1592081 (registered on January 29, 2016)
Korean Patent No. 10-1248668 (registered on March 22, 2013)

### [Disclosure]

### [Technical Problem]

The disclosure has been invented in consideration of the above problems, and an object of the disclosure is to provide a plasma generation module for a uterine cervix that may concentrate plasma generation in a uterine external cervical canal of the uterine cervix.

### [Technical Solution]

According to an embodiment, provided is a plasma generation module for a uterine cervix, the module including: a module body coupled to a front end of a probe part capable of entering a vagina of a human body; a dielectric coupled to an outer front of the module body and facing the uterine cervix while maintaining a distance for a plasma generation space between the dielectric and the uterine cervix; and an electrode installed between the module body and the dielectric and having a shape corresponding to a shape of the dielectric, wherein the dielectric includes a gas discharge part discharging background gas into the plasma generation space.

It is thus possible to maintain optimal position and distance to increase an effect of the plasma energy by providing the plasma generation module having a configuration optimized for a shape of the uterine cervix.

The module may further include a gas supply nozzle coupled through the module body, and supplying the background gas to be moved to the plasma generation space between the electrode and the module body.

It is thus possible to modularize the gas supply nozzle for supplying the background gas and the module body, allowing the same to be easily assembled and installed.

The dielectric may include: a cylindrical dielectric body coupled to the module body; a large-area discharge cover part extending forward from the dielectric body and having a shape corresponding to those of uterine external and internal cervical canals; and a large-area discharge protrusion protruding outward from a center of the large-area discharge cover part and entering a uterine internal cervical canal of the uterine cervix to generate plasma, wherein the gas discharge part protrudes backward from the large-area discharge cover part to pass through the electrode from the outside of the large-area discharge cover part and communicate with the gas supply nozzle.

It is thus possible to provide the dielectric in the shape corresponding to those of the uterine external cervical canal and the uterine internal cervical canal, thereby effectively achieving large-area plasma discharge over an entire area of the uterine cervix.

The large-area discharge cover part may have a round connection part with the dielectric body, and may be gradually depressed from the connection part to the large-area discharge protrusion.

A number of protrusions may protrude from an outer surface of the large-area discharge cover part to secure the plasma generation space by maintaining the distance when the large-area discharge cover part is in contact with the uterine external cervical canal.

It is thus possible to stably maintain a distance between the uterine external cervical canal and the dielectric, thereby effectively generating the plasma energy directly across an entire surface of the uterine external cervical canal.

The module may further include a plasma recovery part recovering the background gas and the plasma between the dielectric and the uterine external cervical canal to the probe part. The plasma recovery part may include: a first plasma recovery line inserted into an outer surface of the module body, and connected to and communicating with the inside of the probe part; and a second plasma recovery line extending from an outer front end of the dielectric to be inserted into its portion in contact with the module body, and connected to the first plasma recovery line.

It is thus possible to increase the effect of the plasma by effectively recovering and treating the background gas and the plasma remaining in the vagina, minimizing heat of a target object that occurs by the plasma, and suppressing a laminar flow on a surface of the target object.

The module body may include: a central cylindrical part disposed approximately in a center of the module body in a length direction, and having a nozzle coupling hole through which the gas supply nozzle is coupled, and an electrode pin coupling hole through which an electrode pin in contact with the electrode is coupled; a first coupling part having a cylindrical shape and extending from one end of the central cylindrical part and coupled to the end of the probe part; and a second coupling part protruding and extending from the other end of the central cylindrical part and coupled to the dielectric.

It is thus possible to simply configure the structure of the plasma generation module, and facilitate its manufacturing and assembly to thus achieve improved productivity.

The electrode may include: a main electrode part having a disk shape corresponding to that of the large-area discharge cover part, and having a through hole through which the gas discharge part passes; and a sub-electrode part protruding from a center of the main electrode part and entering the large-area discharge protrusion.

It is thus possible to provide the shape of the electrode that corresponds to an external shape of the uterine cervix, thus effectively generating the large-area plasma energy directly on the surface of the uterine cervix.

### [Advantageous effect]

The plasma generation module for a uterine cervix according to the disclosure may use the DBD large-area method to generate and provide the plasma energy directly to the surface of the uterine external cervical canal of the uterine cervix.

In particular, the plasma generation module may generate the plasma energy as close to the uterine cervix as possible in order to kill the cancer cell in the uterine cervix.

In addition, the plasma generation module may safely recover the plasma and the background gas, generated close to the uterine external cervical canal, to prevent the plasma and the background gas from leaking to the outside.

In particular, the plasma generation module may ensure that the line to the plasma recovery part is not blocked even when the module is in close contact with the inside of the vagina, thus suppressing the laminar flow and stably recovering the gas and the plasma while maintaining a minute distance between the uterine external cervical canal and the plasma generation module.

In addition, the plasma generation module may have the shapes of the dielectric and the electrode corresponding to the unique shapes of the uterine external and internal cervical canals, the configuration to hold its position, and the protrusion to maintain the distance, thus maintaining an optimal condition in which the plasma energy may act to kill the cancer cell in the uterine external cervical canal.

### [Description of Drawings]

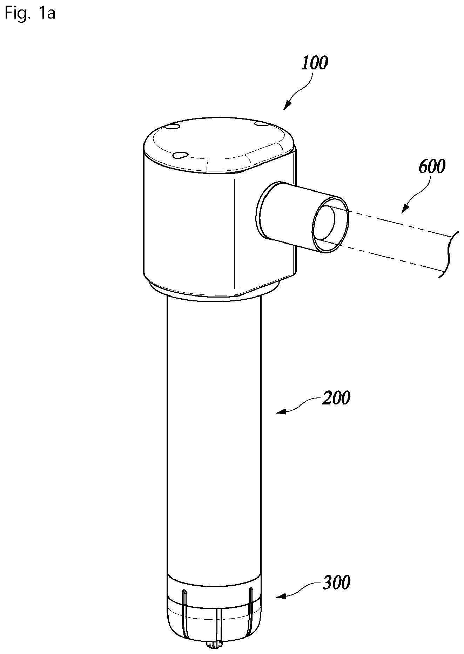

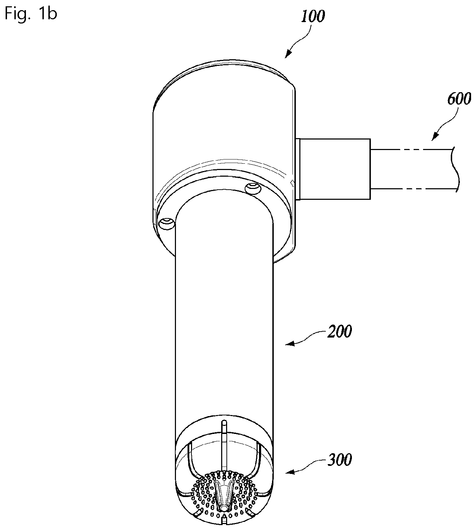

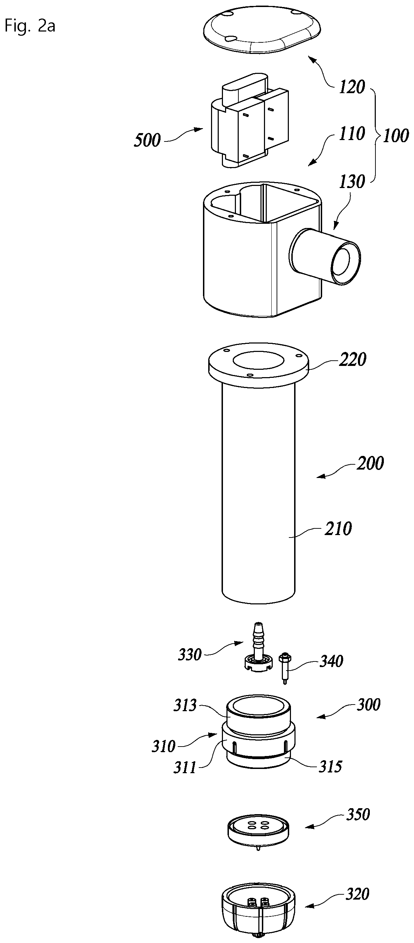

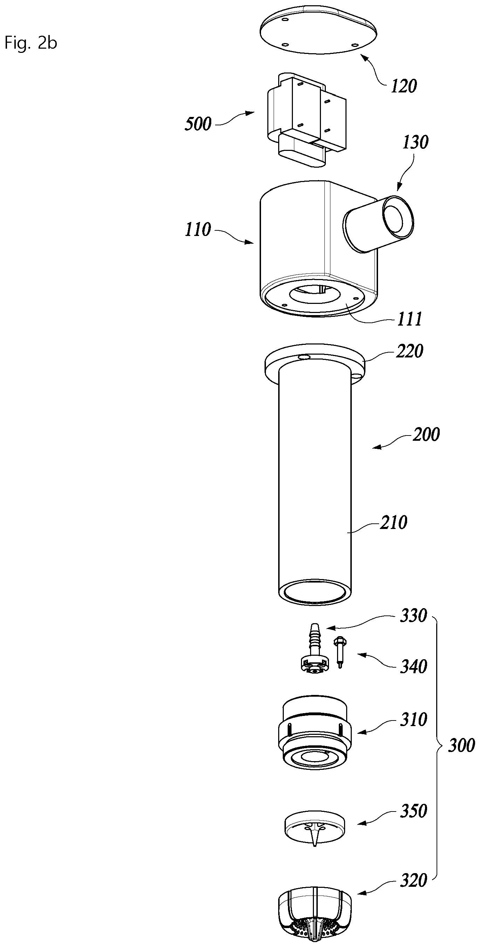

Each of FIGS. 1A and 1B is a perspective view showing a plasma generation device using a plasma generation module for a uterine cervix according to an embodiment of the disclosure.
Each of FIGS. 2A and 2B is an exploded perspective view of the plasma generation device for a uterine cervix shown in FIG. 1A.
FIG. 3 is a front view of the plasma generation device for a uterine cervix shown in FIG. 1A.
FIG. 4 is a plan view of the plasma generation device for a uterine cervix shown in FIG. 1A.
FIG. 5A is a cross-sectional view taken along line I-I of FIG. 4.
FIG. 5B is an enlarged view of part A of FIG. 5A.
FIG. 6 is an exploded cross-sectional view taken along line I-I of FIG. 4.
Each of FIGS. 7 and 8 is an exploded perspective view showing an excerpt of the plasma generation module shown in FIG. 1A.
Each of FIGS. 9A and 9B is a perspective view showing a gas nozzle shown in FIG. 2A.
FIG. 10 is a view to explain a state where plasma is generated by inserting the plasma generation device for a uterine cervix into a vagina according to an embodiment of the disclosure.
FIG. 11 is an enlarged view of part B of FIG. 10.

### [Best Mode]

Hereinafter, a plasma generation module for a uterine cervix according to an embodiment of the disclosure is described in detail with reference to the accompanying drawings.

Each of FIGS. 1A and 1B is a perspective view showing a plasma generation device using a plasma generation module 300 for a uterine cervix according to an embodiment of the disclosure.

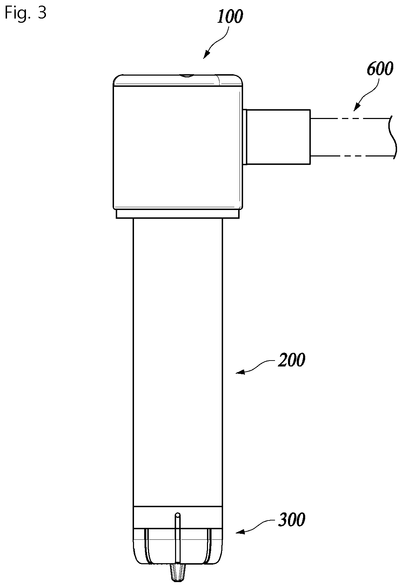

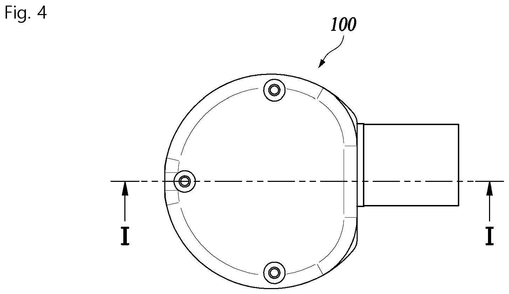

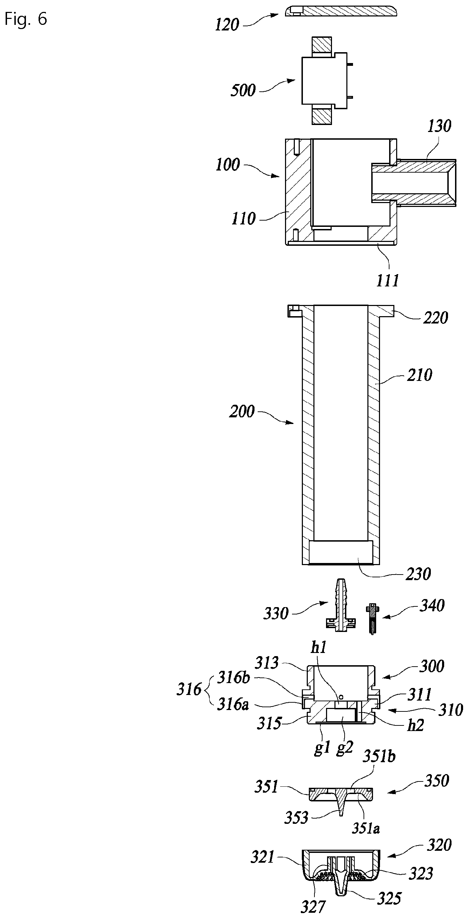

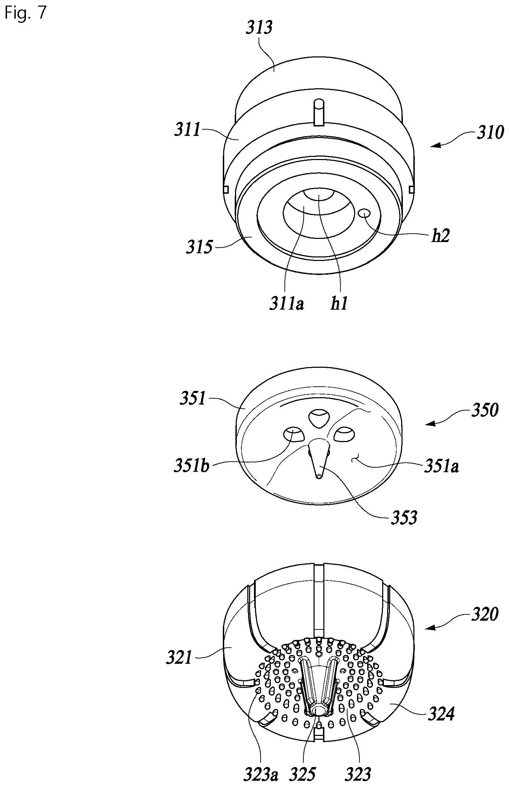

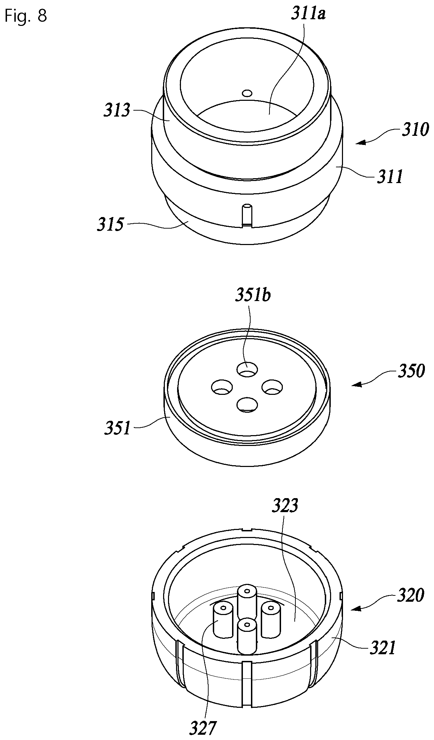

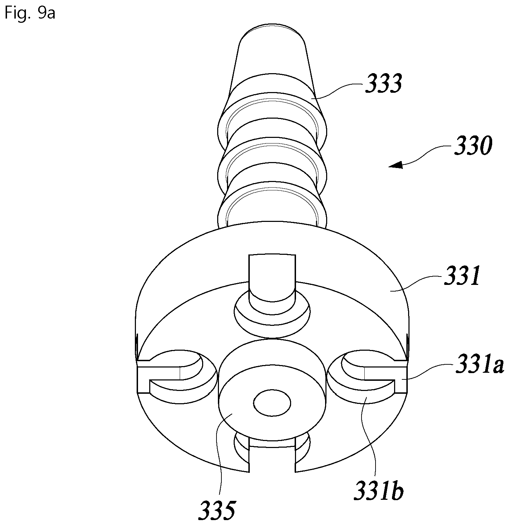

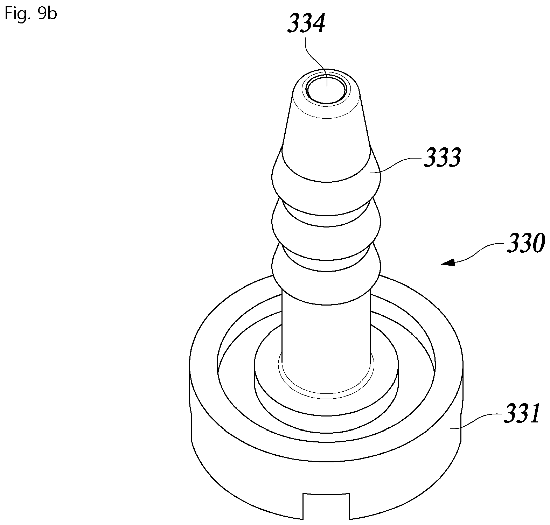

Referring to FIGS. 1A to 11, the plasma generation device for a uterine cervix may include a device body 100, a probe part 200 extending from the device body 100 and capable of entering a vagina of a human body, and a plasma generation module 300 installed at a front end of the probe part 200 and generating plasma by concentrating plasma generation on a surface of a uterine cervix 10 of the human body.

The device body 100 may include a main housing 110 having a transformer 500 installed therein, a housing cover 120 coupled to one end of the main housing 110, a cable support member 130 coupled to a side surface of the main housing 110, and supporting a cable module 600 passing therethrough. The main housing 110 may have both open ends, i.e., one end to which the housing cover 120 is coupled, and the other end to which the probe part 200 is coupled. To this end, a probe coupling part 111 to which the probe part 200 is in close contact with and coupled may be formed at the other end of the main housing 110.

In addition, the transformer 500 may be installed in the main housing 110. The cable support member 130 may be coupled to the side surface of the main housing 110 and have a pipe structure. Therefore, the cable module 600 may be connected by passing from the outside to inside of the main housing 110 through the cable support member 130.

Here, the cable module 600 may include a main power cable 610 for supplying power to the transformer 500, a gas supply hose 620 for supplying background gas to the plasma generation module 300 via the probe part 200, and a plasma suction pipe 630 for recovering the plasma and the gas, which is used by being generated in the plasma generation module 300, by suctioning the same through the probe part 200. The main power cable 610, the gas supply hose 620, and the plasma suction pipe 630 may be provided as a bundle.

In addition, the power converted from the transformer 500 may be provided to the plasma generation module 300 through an electrode pin 340, which is described below, by a sub power cable 640. The transformer 500 may receive the power from the outside, amplify (or convert) the power, and directly provide the amplified power to an electrode 350, which is described below, through the probe part 200. In this way, when the transformer 500 may be installed in the device body 100 installed in the plasma generation module 300, it is possible to acquire the following advantage compared to the prior art where a transformer is installed outside to amplify a voltage and then supply the same. That is, when the transformer is installed outside the device body 100 of the plasma generation device to amplify the voltage and then supply the same, radiation noise due to a high voltage may occur to thus affect a peripheral device, and cause an irregular power loss based on an external circumstance. For example, when twisted into a specific shape, the power cable may have an antenna structure to cause the radiated noise. In addition, when the power cable is close to a ground wire, a strong magnetic field may be formed. In addition, when using the plasma generation device, its output value may be unstable based on a usage environment such as a user movement. To solve this problem, when increasing the output value, electromagnetic wave radiation may become worse.

On the other hand, when the transformer 500 is installed in the device body 100 as in the case of the disclosure, the voltage may be amplified in the device body 100 and provided to the electrode 350 at the shortest distance. As a result, the voltage of a wire may be dramatically lower to minimize radiated power, and the power may be intactly delivered to a source, that is, the electrode 350, without a voltage loss. In particular, a length of the sub power cable 640 connecting the transformer 500 with the electrode pin 340 in the probe part 200 may be set to the shortest distance, the disclosure may not be affected by an external environment and may not cause any movement or transformation, thus solving the electromagnetic wave occurrence, the energy radiation, or the like.

The probe part 200 may have a pipe structure passing through from one end to the other end. The probe part 200 may include a pipe-shaped probe body 210 having a certain outer diameter, and a coupling flange 220 expanding at one end of the probe body 210 and in close contact with and coupled to the probe coupling part 111 of the main housing 110 to maintain airtightness. The probe body 210 may have both open ends, and have appropriate outer diameter and length so that the probe body 210 may be inserted into the vagina of the human body. The coupling flange 220 may expand integrally at one end of the probe body 210, and may be in close contact with and coupled to the probe coupling part 111 of the main housing 110 by a fastening means such as a screw. The plasma generation module 300 may be coupled to the other end of the probe body 210. To this end, an inner coupling part 230 for the coupling of the plasma generation module 300 may be formed on an inner periphery of the other end of the probe body 210, and the inner coupling part 230 may include a thread.

The plasma generation module 300 may include a module body 310 coupled to the inner coupling part 230 of the probe body 210, a dielectric 320 coupled to an outer front of the module body 310, a gas supply nozzle 330 installed in the module body 310, the electrode pin 340, the electrode 350 installed between the module body 310 and the dielectric 320, and the plasma recovery part recovering the background gas supplied between the dielectric 320 and a surface of the uterine cervix 10 and the generated plasma into the probe part 200.

The module body 310 may include a central cylindrical part 311 disposed approximately in its center in a length direction, a first coupling part 313 having a cylindrical shape, extending from one end of the central cylindrical part 311, and coupled to the inner coupling part 230 of the probe body 210, and a second coupling part 315 protruding and extending from the other end of the central cylindrical part 311 and coupled to the dielectric 320. The central cylindrical part 311 may have an outer diameter corresponding to the outer diameter of the probe body 210, and have a partition 311a formed therein. The partition 311a may block the inside of the module body 310 between the first and second coupling parts 313 and 315. In addition, a nozzle coupling hole h1 through which the gas supply nozzle 330 is coupled may be formed in the center of the partition 311a. In addition, an electrode pin coupling hole h2 through which the electrode pin 340 is fitting-coupled may be formed in the partition 311a to communicate with an end of a second coupling part 314. Each of the first and second coupling parts 313 and 315 may have an outer diameter smaller than that of the central cylindrical part 311. Therefore, the first coupling part 313 may be inserted into and screw coupled to the inner coupling part 230 of the probe body 210, and the dielectric 320 may be coupled to the second coupling part 315. An annular sealing groove to which a sealing ring is coupled may be inserted into an outer periphery of the first coupling part 313.

In addition, a first inlet groove g1 may be formed concentrically with the nozzle coupling hole h1 while having a predetermined depth from an end of the second coupling part 315, and a second insertion groove g2 may be inserted concentrically with the nozzle coupling hole h1 in the bottom of the first insertion groove g1. The second insertion groove g2 may communicate with the nozzle coupling hole h1. The electrode pin coupling hole h2 may pass through the partition wall 311a to be exposed and extending to the outside of the first insertion groove g1. Therefore, the electrode pin 340 coupled to the electrode pin coupling hole h2 may be mounted to protrude into the first insertion groove g1.

In addition, a plurality of first plasma recovery lines 316 of the plasma recovery part may be formed in an outer periphery of the central cylindrical part 311. The first plasma recovery line 316 may be formed at a certain distance in the outer periphery of the central cylindrical part 311 in a circumferential direction. The first plasma recovery line 316 may include a plasma recovery groove 316a inserted by a predetermined length into the outer periphery of the central cylindrical part 311, and a plasma recovery hole 316b connected to one end of the plasma recovery groove 316a and communicating with the inner space of the first coupling part 313. The plasma recovery groove 316a may start from its boundary with the second coupling part 315 of the central cylindrical part 311 and extend to its position past the partition 311a of the central cylindrical part 311. The plasma recovery hole 316b may connect the plasma recovery groove 316a with the inner space of the first coupling part 313, that is, the inner space of the probe body 210.

The dielectric 320 may be coupled to the second coupling part 315 of the module body 310, thus concentrating the plasma generation in a large area on the surface of the uterine cervix 10, that is, over a uterine external cervical canal 11 and a uterine internal cervical canal 12.

The dielectric 320 may include a cylindrical dielectric body 321 coupled to the second coupling part 315, a large-area discharge cover part 323 extending forward from the dielectric body 321 and having a shape corresponding to that of the uterine external cervical canal 11, a large-area discharge protrusion 325 protruding outward from the center of the large-area discharge cover part 323 and disposed toward the uterine internal cervical canal 12 of the uterine cervix 10 to generate the plasma, and a gas discharge part 327 moving the background gas supplied by the gas supply nozzle 330 between the module body 310 and the electrode 350 to a plasma generation space between the large-area discharge cover part 323 and the uterine cervix 10.

A coupling hook 321a protruding inward may be formed at an end of the dielectric body 321 so that the dielectric body 321 may be coupled to the outside of the second coupling part 315 in a so-called one-touch type. To this end, an annular coupling groove to which the coupling hook 321a is coupled may be formed in an outer surface of the second coupling part 315.

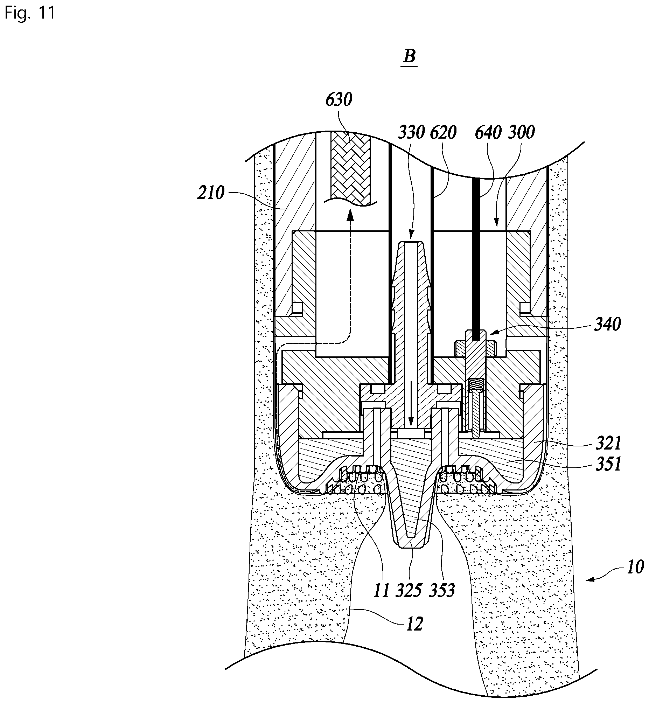

A round connection part 324 may be provided between the dielectric body 321 and the large-area discharge cover part 323. The large-area discharge cover part 323 may be gradually depressed from the connection part 324 to the center. Accordingly, as shown in FIG. 11, the large-area discharge cover part 323 may be disposed to surround the uterine external cervical canal 11 while maintaining a certain distance therefrom. A number of protrusions 323a for maintaining the distance may preferably protrude from an outer surface of the large-area discharge cover part 323. By the protrusion 323a for maintaining the distance, it is possible to maintain an optimal distance, that is, the plasma generation space which allows the plasma energy to be effectively generated between the large-area discharge cover part 323 and the uterine external cervical canal 11, and the plasma may be generated in a minute distance.

In addition, a large-area discharge protrusion 325 may protrude from the center of the large-area discharge cover part 323. The large-area discharge protrusion 325 may have a conical shape having an outer diameter decreased toward its end. In addition, a rib 325a for maintaining the distance may preferably be formed on an outer surface of the large-area discharge protrusion 325 in a length direction of the large-area discharge protrusion 325. The rib 325a for maintaining the distance may be formed at a certain distance on the outer surface of the large-area discharge protrusion 325 in the circumferential direction to maintain the optimal distance for the plasma to be effectively generated by resonance between the large-area discharge protrusion 325 and the uterine internal cervical canal 12.

The large-area discharge protrusion 325 having this configuration may be disposed at an entrance of the uterine internal cervical canal 12, thus serving as a guide the dielectric 320 to be disposed precisely in the center of the uterine cervix and stably maintain its posture. In addition, by the rib 325a for maintaining the distance, it is possible to maintain the distance for effectively achieving the plasma generation by the resonance between the large-area discharge protrusion 325 and the uterine internal cervical canal 12. Therefore, even in the internal cervical canal 12, the plasma generation may be achieved between a surface of the uterine internal cervical canal, which is a target object, and the large-area discharge protrusion 325 by using the DBD method.

The plurality of gas discharge parts 327 may be disposed around the large-area discharge protrusion 325. The gas discharge part 327 may protrude inward from the large-area discharge cover part 323, and may protrude while having a length that allows the gas discharge part to pass through a through hole 351b of the electrode 350, which is described below, and enter the second insertion groove g2 of the second coupling part 315. The gas discharge part 327 may pass through a boundary between the large-area discharge cover part 323 and the large-area discharge protrusion 325, thus supplying the gas to the plasma generation space between the uterine cervix 10 and the dielectric 320. An end of the gas discharge part 327 may be coupled to a seating groove 331b of the gas supply nozzle 330.

In addition, a second plasma recovery line 328 of the plasma recovery part may be formed in an outer surface of the dielectric 320. The second plasma recovery line 328 may include a slit extending from the outer surface of the large-area discharge cover part 323 to the end of the dielectric body 321. The plurality of second plasma recovery lines 328 may be formed at a position connected to the first plasma recovery line 316.

The dielectric 320 having the above configuration may allow a space to be secured by maintaining its minute distance to the external cervical canal 11, and the gas to be supplied to the secured space. Therefore, when the power is supplied to the electrode 350 disposed in the dielectric 320, it is possible to generate the plasma in the best condition in the plasma generation space between the dielectric 320 and the uterine cervix 10 which is the target object.

In addition, as described above, it is possible to recover the plasma and the gas, generated in the space between the dielectric 320 and the uterine cervix 10 by suctioning the same into the probe part 200. Therefore, the plasma generation space may maintain a certain distance without being expanded more than necessary by the gas, and heat of the target object (or the uterine cervix) that occurs by the plasma may be minimized as the gas is recovered in real time to exchange the heat.

The gas supply nozzle 330 is for supplying the background gas to a space between the module body 310 and the electrode 350. The gas supply nozzle 330 may have a gas nozzle body 331 mounted in the second insertion groove g2 of the module body 310, a hose coupling part 333 protruding from one surface of the gas nozzle body 331, and a gas discharge part 335 protruding from the other end of the gas nozzle body 331. The gas nozzle body 331 may have a disk shape, and may be inserted into and mounted in the second insertion groove g2. The hose coupling part 333 may protrude from the center of one surface of the gas nozzle body 331. The hose coupling part 333 may be formed coaxially with the gas discharge part 335 protruding from the center of the other end of the gas nozzle body 331 and share a nozzle hole 332.

In addition, a plurality of gas inlet holes 331a may be formed in an outer surface of the gas nozzle body 331. The plurality of gas inlet holes 331a may be formed at a certain distance in an outer peripheral surface of the gas nozzle body 331 in the circumferential direction. In addition, the seating groove 331b to which the gas discharge part 327 is coupled may be inserted in the other surface of the gas nozzle body 331. The plurality of seating grooves 331b may communicate with the gas inlet hole 331a. Accordingly, the gas supplied through the gas supply nozzle 330 may be introduced into the gas discharge part 327 and supplied between the target object and the dielectric 320.

The hose coupling part 333 may be disposed in the probe part 200 while protruding by a predetermined length from the center of one surface of the gas nozzle body 331. The gas supply hose 620 may be coupled to the hose coupling part 333. The gas supply hose 620 may be included in the cable module 600, may enter the probe part 200, and may be coupled to the hose coupling part 333, and the background gas may thus be supplied through the gas supply nozzle 330.

The gas discharge part 335 may protrude from the other end of the gas nozzle body 331, and discharge the background gas supplied through the gas supply hose 620 coupled to the hose coupling part 333.

The gas supply nozzle 330 having the above configuration may be coupled to the module body 310 so that the hose coupling part 333 passes through the nozzle coupling hole h1 and protrudes into the first coupling part 313.

The electrode pin 340 may be coupled to the electrode pin coupling hole h2 of the module body 310. The electrode pin 340 may include a pin body 341 fixedly coupled thereto by passing through the electrode pin coupling hole h2, a contact pin 343 installed to protrude from a tip of the pin body 341 and in contact with the electrode 350, an elastic member 345 elastically pressing the contact pin 343 to protrude from the tip of the pin body 341. The elastic member 345 may be installed in the pin body 341, and elastically press the contact pin 343 to stably maintain its contact with the electrode 350. The electrode pin 340 may be connected to the transformer 500 by the sub power cable 640 to directly receive the amplified power.

The electrode 350 may include a main electrode part 351 having a disk shape corresponding to that of the large-area discharge cover part 323, and a sub-electrode part 353 protruding from the center of the main electrode part 351 and disposed in the large-area discharge protrusion 325. A depression part 351a depressed deeper than an edge may be formed in a front surface of the main electrode part 351 that is in contact with the dielectric 320 to be in contact with the large-area discharge cover part 323. In addition, the through hole 351b may be formed in the main electrode part 351, and the gas discharge part 327 may pass through and be coupled to the through hole 351b. The sub-electrode part 353 may have a shape corresponding to that of the large-area discharge protrusion 325, and protrude from the center of the main electrode part 351. In this way, the sub-electrode part 353 may protrude and couple into the large-area discharge protrusion 325, thus allowing the plasma to be effectively generated by the resonance in a space between the large-area discharge protrusion 325 and the uterine internal cervical canal 12.

The electrode 350 having the above configuration may be interposed between the module body 310 and the dielectric 320 to maintain its electrical insulation from the outside.

Hereinafter, the description describes in detail an effect of the plasma generation module for a uterine cervix that has the above configuration according to an embodiment of the disclosure.

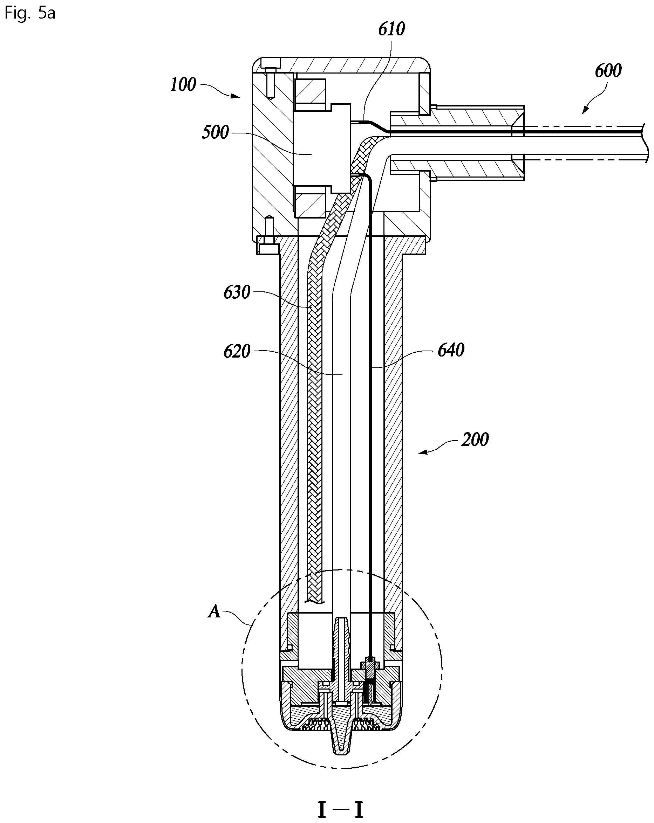

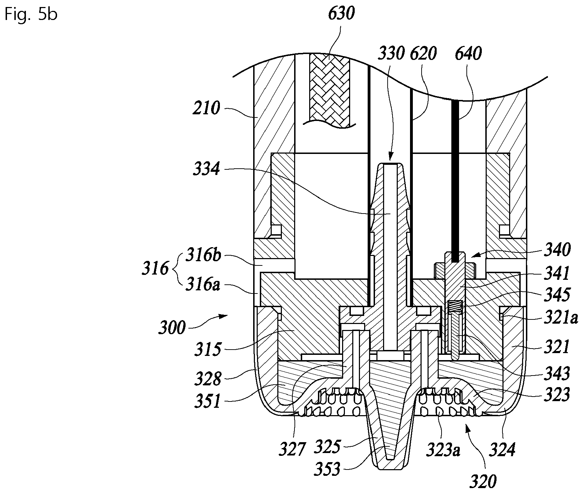

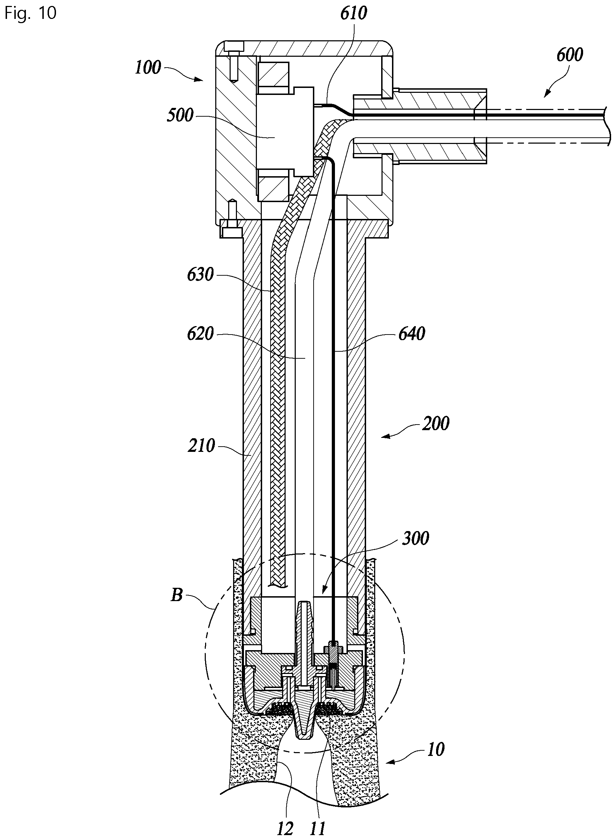

First, the assembled plasma generation device for a uterine cervix as shown in FIGS. 5A and 5B may be used to generate the plasma energy in the uterine cervix, thereby providing a therapeutic help to a patient with the uterine cervix precancerous lesion. To this end, the probe part 200 may be allowed to enter the vagina of a uterus. Here, as shown in FIGS. 10 and 11, when the probe part 200 sufficiently enters the vagina, the dielectric 320 of the plasma generation module 300 may be disposed in close contact with the uterine external cervical canal 11. In particular, the large-area discharge protrusion 325 may be disposed to enter the entrance of the uterine internal cervical canal 12, thus accurately disposing the position of the dielectric 320 with respect to the uterine external cervical canal 11. In addition, an outer shape of the dielectric 320 and the protrusion 323a protruding outward therefrom may prevent the dielectric 320 from coming into close contact with the uterine external cervical canal 11 and secure the optimal space where the plasma energy may be generated and act thereon effectively. That is, the plasma generation module 300 of the disclosure may use the direct large-area discharge by the DBD method. Therefore, the closer the distance of the plasma generation module 300 to the target object, the easier the plasma generation. However, when the target object and the dielectric 320 come into complete contact with each other, a resonance region may disappear to suppress the plasma generation. When the protrusion 323a is formed on the surface of the large-area discharge cover part 323, it is possible to maintain the certain distance of the plasma generation module 300 to the target object (or the uterine cervix). Therefore, the plasma generation may be performed effectively by preventing the contact between the target object and the dielectric 320.

In addition, the protrusion 323a disposed on the dielectric 320 may maintain the distance between the dielectric 320 and the target object. It is thus possible to supply the gas to thus secure plasma discharge stability and move a fluid quickly between the small protrusions 323a, thereby suppressing a laminar flow on the surface of the target object and perform rapid heat exchange. In this way, it is possible to minimize the heat of the target object that occurs by the plasma.

Meanwhile, when the probe part 200 enters the vagina, and the plasma generation device is activated, the power amplified and converted by the transformer 500 may be supplied to the electrode 350 through the electrode pin 340. In addition, the background gas may be supplied to the gas supply nozzle 330 and the gas discharge part 327 through the gas supply hose 620 to be supplied to the plasma generation space, that is, a micro-space between the dielectric 320 and the target object (or the uterine cervix). The resonance may then be generated in the plasma generation space and the plasma energy is discharged and generated. Here, the generated plasma energy may act on the surfaces of the uterine external cervical canal 11 and the uterine internal cervical canal 120. As a result, the plasma energy may effectively act to kill the cancer cell in the uterine external cervical canal 11 and the uterine internal cervical canal 120. That is, the dielectric 320 may surround the surface of uterine cervix 10 while maintaining the certain distance, and directly generate the plasma through the resonance in the plasma generation space therebetween, thus efficiently transmitting its electrical energy, ion energy, optical (or physical) effect, reactive oxygen species (ROS) and reactive nitrogen species (RNS). As described above, when the dielectric 320 and the uterine cervix 10 maintain the certain distance therebetween, a large area of the uterine cervix may be effectively treated with the same voltage and a low flow rate regardless of a treatment area, thus increasing a plasma processing effect while reducing power consumption. In addition, in this way, it is possible to lower manufacturing costs by increasing plasma generation efficiency and processing efficiency without increasing a capacity of the transformer 500.

In addition, the background gas and the plasma staying in the plasma generation space between the uterine cervix 10 and the dielectric 320 may be recovered by being suctioned into the probe part 200 via the second plasma recovery line 328 and the first plasma recovery line 316. Accordingly, it is possible to maintain the certain distance for the plasma generation space, and rapidly exchange the heat to thus minimize the heat of the uterine cervix 10 that occurs by the plasma.

The background gas and the plasma, suctioned into the probe part 200, may be processed by being recovered to the outside through the suction pipe 630. Here, even though the outer surface of the dielectric 320 is in close contact with an inner wall of the vagina, each of the second plasma recovery line 328 and the first plasma recovery line 316 may have a slit structure, thus effectively recovering the background gas and the plasma while preventing blockage of the plasma recovery line.

The plasma generation module for a uterine cervix that has the above configuration may have a configuration optimized for a structure in the vagina and a unique shape of the uterine cervix. That is, the disclosure may have a configuration in which the dielectric 320 of the plasma generation module 300 may maintain the optimal distance allowing the plasma energy to be generated and act in the uterine external cervical canal 11 and uterine internal cervical canal 12 of the uterine cervix 10. As a result, the plasma energy may be effectively generated to kill the cancer cell in the uterine external cervical canal 11 and uterine internal cervical canal 12 of the uterine cervix.

Although the disclosure is shown and described with respect to the specific embodiment hereinabove, it is apparent to those skilled in the art that the disclosure may be variously modified and altered without departing from the spirit and scope of the disclosure. Therefore, these modifications and alterations should be considered to be included within the spirit and scope of the disclosure as defined by the following claims.

### [Industrial Applicability]

The disclosure may provide a plasma generation module for a uterine cervix that may concentrate plasma generation in both the external and internal cervical canals of a uterine cervix transformation zone where a uterine cervix precancerous lesion occurs.

## Claims

1. A plasma generation module for a uterine cervix, the module comprising:
a module body coupled to a front end of a probe part capable of entering a vagina of a human body;
a dielectric coupled to an outer front of the module body and facing the uterine cervix while maintaining a distance for a plasma generation space between the dielectric and the uterine cervix; and
an electrode installed between the module body and the dielectric and having a shape corresponding to a shape of the dielectric,
wherein the dielectric includes a gas discharge part discharging background gas into the plasma generation space.

2. The module of claim 1, further comprising a gas supply nozzle coupled through the module body, and supplying the background gas to be moved to the plasma generation space between the electrode and the module body.

3. The module of claim 1, wherein the dielectric includes:
a cylindrical dielectric body coupled to the module body;
a large-area discharge cover part extending forward from the dielectric body and having a shape corresponding to that of a uterine external cervical canal; and
a large-area discharge protrusion protruding outward from a center of the large-area discharge cover part and entering a uterine internal cervical canal of the uterine cervix to generate plasma,
wherein the gas discharge part protrudes backward from the large-area discharge cover part to pass through the electrode from the outside of the large-area discharge cover part and communicate with the gas supply nozzle.

4. The module of claim 3, wherein the large-area discharge cover part has a round connection part with the dielectric body, and is gradually depressed from the connection part to the large-area discharge protrusion.

5. The module of claim 3, wherein a number of protrusions protrude from an outer surface of the large-area discharge cover part to secure the plasma generation space by maintaining the distance when the large-area discharge cover part is in contact with the uterine external cervical canal.

6. The module of any one of claims 1 to 5, further comprising a plasma recovery part recovering the background gas and the plasma between the dielectric and the uterine external cervical canal to the probe part.

7. The module of claim 6, wherein the plasma recovery part includes:
a first plasma recovery line inserted into an outer surface of the module body, and connected to and communicating with the inside of the probe part; and
a second plasma recovery line extending from an outer front end of the dielectric to be inserted into its portion in contact with the module body, and connected to the first plasma recovery line.

8. The module of any one of claims 1 to 5, wherein the module body includes:
a central cylindrical part disposed approximately in a center of the module body in a length direction, and having a nozzle coupling hole through which the gas supply nozzle is coupled, and an electrode pin coupling hole through which an electrode pin in contact with the electrode is coupled;
a first coupling part having a cylindrical shape and extending from one end of the central cylindrical part and coupled to the end of the probe part; and
a second coupling part protruding and extending from the other end of the central cylindrical part and coupled to the dielectric.

9. The module of any one of claims 3 to 5, wherein the electrode includes:
a main electrode part having a disk shape corresponding to that of the large-area discharge cover part, and having a through hole through which the gas discharge part passes; and
a sub-electrode part protruding from a center of the main electrode part and entering the large-area discharge protrusion.
